# FASCICULE DE BREVET EUROPEEN

(11) **EP 3 003 263 B1**
(45) Date de publication et mention de la délivrance du brevet: **14.11.2018**
(21) Numéro de dépôt: 14727825.3
(22) Date de dépôt: 03.06.2014
(51) Int. Cl.: A61P 35/00, A61P 17/00, A61K 8/64, A61K 8/67, A61K 31/11, A61K 38/05, A61Q 19/08, A61Q 19/00, A61K 31/7048

(54) **COMPOSITIONS COSMETIQUES OU DERMATOLOGIQUES ASSOCIANT DU RETINALDEHYDE ET DE L'OLEAMIDE DE GLYCYLGLYCINE, ET LEURS UTILISATIONS EN COSMETIQUE OU DERMATOLOGIE**
KOSMETISCHE ODER DERMATOLOGISCHE ZUSAMMENSETZUNGEN MIT RETINALDEHYD UND GLYCYL-GLYCIN-OLEAMID SOWIE KOSMETISCHE ODER DERMATOLOGISCHE VERWENDUNGEN DAVON
COSMETIC OR DERMATOLOGICAL COMPOSITIONS COMBINING RETINALDEHYDE AND GLYCYLGLYCINE OLEAMIDE, AND THE COSMETIC OR DERMATOLOGICAL USES THEREOF

(30) Priorité: 03.06.2013 FR 1355036
(43) Date de publication de la demande: 13.04.2016
(73) Titulaire: Pierre Fabre Dermo-Cosmétique, 92100 Boulogne-Billancourt (FR)
(72) Inventeur: BACQUEVILLE, Daniel, F-31100 Toulouse (FR); BOGDANOWICZ, Patrick, F-31130 Balma (FR); SAURAT, Jean-Hilaire, CH-1206 Genève (CH); SORG, Olivier, CH-1202 Genève (CH)
(74) Mandataire: Regimbeau
(86) Numéro de dépôt international: PCT/EP2014/061488
(87) Numéro de publication internationale: WO 2014/195315

(56) Documents cités:
- EP-A1- 1 061 897
- EP-A1- 1 062 223
- BOGDANOWICZ P ET AL: "Anti-aging properties of the association of "Retinaldehyde, Pretocopheryl glucopyranosyl, Glycylglycine oleamide"", JOURNAL OF INVESTIGATIVE DERMATOLOGY, vol. 133, no. Suppl. 1, mai 2013 (2013-05) , page S150, XP002715765, & INTERNATIONAL INVESTIGATIVE DERMATOLOGY MEETING; EDINBURGH, UK; MAY 08 -11, 2013
- BOISNIC S ET AL: "Comparative study of the anti-aging effect of retinaldehyde alone or associated with pretocopheryl in a surviving human skin model submitted to ultraviolet A and B irradiation", INTERNATIONAL JOURNAL OF TISSUE REACTIONS, vol. 27, no. 3, 2005, pages 91-99, XP009173915, ISSN: 0250-0868
- OLIVIER SORG ET AL: "Retinoids in cosmeceuticals", DERMATOLOGIC THERAPY, MUNKSGAARD, COPENHAGEN, DK, vol. 19, no. 5, 2 octobre 2006 (2006-10-02), pages 289-296, XP002718240, ISSN: 1396-0296, DOI: 10.1111/J.1529-8019.2006.00086.X [extrait le 2006-10-02] cité dans la demande

## Description

La présente invention a pour objet des compositions topiques à base de rétinaldéhyde et d'oléamide de glycylglycine et le cas échéant de delta-tocophéryl-glucopyranoside, leur utilisation en cosmétologie et en dermatologie.

La peau doit sa souplesse et sa résistance aux différentes couches de tissus qui la composent : l'épiderme, le derme et l'hypoderme. L'épiderme, couche extérieure de la peau est responsable de son imperméabilité et de sa résistance. Il est essentiellement composé de kératine, une protéine fibreuse produite par les kératinocytes et de mélanine, le principal pigment cutané fabriqué par les mélanocytes. Avec les années, le renouvellement des kératinocytes s'effectue plus lentement et leur différentiation terminale est ralentie. Au cours du temps, de profondes modifications se produisent au niveau du derme. Tissu de soutien de la peau, il est constitué à 80% de fibres d'élastine et de collagène noyées dans un gel de glycoprotéines. Les fibroblastes qui sont les principales cellules du derme, sont spécialisés dans la synthèse de ces fibres d'élastine et de collagène. Entre 20 et 80 ans, la population de fibroblastes diminue de moitié. Ils assurent l'équilibre entre synthèse, maturation et dégradation des fibres d'élastine et de collagène. Dans le temps, cet équilibre va se déplacer vers une dégradation de ces fibres avec pour résultats, une perte d'élasticité et de tonicité du derme et une flaccidité qui ne s'oppose plus aux effets de contraction des muscles sous-jacents, conduisant à l'apparition de rides. Les fibres de collagène sont réparties dans toutes les couches du derme, elles sont capables de fixer l'eau et contribuent à l'hydratation de la peau. Une diminution du collagène et/ou une modification de sa qualité conduisent à l'apparition de rides profondes. Avec le vieillissement, les fibres d'élastine qui confèrent élasticité et solidité aux tissus, se raréfient, la peau devient plus mince et se ride. La peau doit continuellement faire face à de multiples agressions extérieures qui peuvent accélérer le processus naturel du vieillissement. Elle est particulièrement sensible aux attaques des radicaux libres générés à la fois par le fonctionnement normal de notre organisme et par des éléments extérieurs, tels la pollution, le tabac, mais surtout le rayonnement solaire. Ces radicaux libres sont responsables de modifications tissulaires et cellulaires qui conduisent au vieillissement cutané.

La vitamine A (rétinol) et ses dérivés naturels et synthétiques, collectivement désignés comme rétinoïdes, constituent une grande variété de substances ayant des effets marqués, notamment dans l'embryogénèse, la reproduction, la vision, la croissance, l'inflammation, la différenciation, la prolifération et l'apoptose. Les effets biologiques des rétinoïdes sont médiés par leur interaction avec les récepteurs nucléaires du type RAR (récepteur de l'acide rétinoïque) et RXR (récepteur X de l'acide rétinoïque). Le ligand connu des récepteurs RARs est l'isomère trans de la forme acide de la vitamine A.

La peau contient des quantités significatives de rétinoïdes, ainsi que les enzymes impliquées dans leur métabolisme. Les rétinoïdes sont des dérivés naturels ou synthétiques de la vitamine A. Comme rétinoïdes naturels, on peut citer le rétinol et tous ses dérivés métaboliques, le rétinaldéhyde, l'acide tout trans-rétinoïque, l'acide 9-cis rétinoïque, l'acide 13-cis rétinoïque ainsi que ses esters tels que le palmitate de rétinyle. Les rétinoïdes de synthèse sont des molécules dont une portion a été modifiée dans le but d'avoir des composés ayant un seul effet relatif à la vitamine A. Les principales molécules sont l'étrétinate, l'acitrétine, le tazarotène, l'adapalène, le bexarotène, le motrétinide.

L'épiderme humain contient deux formes principales de vitamine A, le rétinol et les esters rétinyliques. La vitamine A est stockée dans les kératinocytes par l'estérification du rétinol en esters rétinyliques. Cette étape est catalysée par deux enzymes, l'acyl CoA acyltranférase et la lécithine rétinol acyltransférase. Leur expression est modulée par les radiations UV et l'état de différentiation des kératinocytes. L'hydrolyse des esters rétinyliques en rétinol est catalysée par la rétinyl ester hydrolase. D'un autre côté, le rétinol est une pro-hormone de l'acide rétinoïque, il est oxydé en rétinaldéhyde qui est lui-même oxydé en acide rétinoïque, forme biologiquement active de la vitamine A. Les esters rétinyliques sont considérés comme la forme de stockage de la vitamine A, étant donné que leurs concentrations dans l'épiderme, et principalement dans le stratum corneum, sont plus élevées que dans le derme ou encore dans le sang et qu'ils sont les précurseurs des autres formes actives de vitamine A. La nature chimique des rétinoïdes, comprenant une chaîne lipidique polyinsaturée, de même que leur propriétés physiques leur conférant un maximum d'absorption compris entre 320 et 390 nm, les rend capables d'interagir avec les UV, ou avec l'oxygène pour produire des formes réactives d'oxygène ou des radicaux libres. Ils peuvent être isomérisés par les UV. Mais dans le plus grand nombre de cas, les rétinoïdes sont purement et simplement détruits sous l'action des UV. Il semblerait que les esters rétinyliques soient plus sensibles que le rétinol. L'épiderme exposé au soleil contient moins d'esters rétinyliques que la peau non exposée, indiquant que les UV induisent une déficience en vitamine A (Sorg et al., Dermatology, 199(4) : 302-307, 1999). Ceci, ainsi que d'autres observations, supportent l'hypothèse selon laquelle la déplétion en vitamine A, induite par le soleil, est impliquée dans la pathogenèse de certains cancers de la peau ainsi que de son vieillissement prématuré (Sorg et al., Dermatol. Ther., 19(5) : 289-296, 2006). Cette déplétion pourrait être compensée par l'apport externe de vitamine A.

L'application topique de rétinoïdes est connue pour apporter de la vitamine A à la peau. Il est connu par ailleurs que l'un des effets secondaires majeurs des rétinoïdes lors d'une application topique reste l'irritation induite. Cet effet peut-être rédhibitoire à l'observance du traitement.

Le rétinol topique exerce des effets biologiques similaires mais moins intenses à ceux de l'acide rétinoïque topique, avec cependant moins d'irritation, ces réponses sont médiées par la conversion du rétinol en acide rétinoïque. Le rétinol, ainsi que ses esters doivent impérativement être convertis en acide rétinoïque pour induire des effets biologiques dans les kératinocytes humains. De la même manière, le rétinaldéhyde qui est converti à la fois en forme de stockage (esters rétinyliques) et en forme active (acide rétinoïque), exerce des activités biologiques significatives et est mieux toléré que l'acide rétinoïque (Saurat et al., J. Invest. Dermatol., 103 : 770-774, 1994). Il est aussi un bon candidat pour l'apport de vitamine A dans l'épiderme. Il a été démontré que le rétinaldéhyde exerce une activité biologique dans la peau en induisant une hyperplasie épidermique, de même qu'une nette augmentation de l'expression du CD44 et du hyaluronate dans l'épiderme folliculaire et interfolliculaire des souris C57BL/6 et SKH1 sans poils (souris hairless). Ces effets ont également été observés suite à l'application topique de l'acide rétinoïque et du rétinol. Toutefois, l'expression du CD44 et celle du hyaluronate ont été plus fortement augmentées chez les souris traitées au rétinaldéhyde. Le CD44 est une glycoprotéine transmembranaire polymorphiques qui a plusieurs isoformes générées par l'épissage alternatif et les modifications post-traductionnelles. Il a été démontré que deux fonctions majeures du CD44 dans la peau murine sont 1) la régulation de la prolifération kératinocytaire en réponse à des stimuli extracellulaires et 2) le maintien de l'homéostasie locale du hyaluronate (Kaya et al., Genes & Development, 11(8) : 996-1007, 1997). Il a également été observé une diminution de l'expression du CD44 épidermique chez les patients souffrant de lichen scléro-atrophique, qui est potentiellement responsable de la déposition dermique du hyaluronate et de l'atrophie épidermique dans cette maladie (Kaya et al., J. nvest. Dermatol., 115(6) : 1054-1058, 2000).

L'application des rétinoïdes se fait selon deux stratégies en dermatologie :
- à des doses pharmacologiques dans le cas de traitement de pathologies cutanées, l'application d'acide rétinoïque (gels ou crèmes de 0,025% à 0,1%) a pour indication principale le traitement de l'acné. Elle s'utilise aussi dans le traitement du photo vieillissement et des kératoses actiniques. Le tazarotène est indiqué dans le traitement du psoriasis (gels à 0,05% à 0,1%). Le motrétinide (crème ou lotion à 0,1%) est quant à lui indiqué dans le traitement de l'acné légère.
- A des fins cosmétiques : le palmitate de rétinyle (gels de 0,5% à 5%), le rétinol (0,1% à 0,6%) et le rétinaldéhyde (0,05%) sont utilisés pour les traitements anti-âges et soins réparateurs.

Les rayonnements solaires incluent la lumière visible, les ultraviolets (UV), les infrarouges et d'autres radiations électromagnétiques. De même que la lumière visible peut être divisée en plusieurs couleurs, les UV peuvent être classifiés en trois subdivisions :
- les UVA (315-400 nm) représentent la part la plus importante d'UV qui atteint la surface de la terre. Ils provoquent sur la peau le bronzage et peuvent l'endommager en profondeur à cause de leur facilité à pénétrer les tissus.
- Les UVB (280-315 nm) représentent une plus petite proportion de rayons atteignant le sol, mais ont une contribution importante aux effets biologiques consécutifs aux expositions solaires. Ils peuvent provoquer rougeurs, brûlures, bronzage, lésions oculaires. Leur pénétration dans la peau est plus faible.
- Les UVC (200-280 nm) sont pratiquement entièrement absorbés par la couche d'ozone de l'atmosphère. Ils ne pénètrent que peu dans la peau mais peuvent provoquer des lésions oculaires.

Les principaux effets des UV sur la peau sont l'érythème la photosénescence, le cancer de la peau. Après exposition aux UV, il y a une augmentation de l'expression des métalloprotéinases dans l'épiderme et le derme. Ces enzymes qui altèrent la production de collagène dans le derme contribuent à l'apparition de rides. L'induction de ces enzymes est maintenue par des expositions répétées (Fisher et al., N. Engl. J. Med., 337 : 1419-1428, 1997). Il y a un épaississement du derme dû à l'accumulation de matériel élastique. La production d'interleukines par l'épiderme, suite à une irradiation UV, contribue à la destruction du tissu conjonctif. Il semblerait que les effets observés dans le derme soient attribuables aux UVA, qui pénètrent jusque-là, contrairement aux UVB. Chez la souris hairless, comme chez l'homme, on observe l'apparition d'hyperplasie et de rides après irradiation à long terme par les UVB, et la peau devient flasque sous l'action des UVA. Les effets observés dépendent de la longueur d'onde des irradiations. Le derme s'épaissit, après irradiation avec des longueurs d'ondes comprises entre 295 et 300 nm, les fibres de collagène sont endommagées et il y a production de fibres élastiques anormales, on parle d'élastose. Après 10 semaines d'irradiation avec des UVA, il n'y a plus de fibres élastiques normales.

De nombreuses études ont permis d'établir un lien entre exposition solaire chronique et prédisposition à développer des cancers de la peau. De plus, les UV modifient certains gènes tels que le p53 responsable de l'élimination ou de la réparation des lésions au niveau de l'ADN. Les UVB et les UVA n'ont pas le même effet sur l'immunité cellulaire chez l'homme. Les UVA sont moins carcinogènes. Les UVB jouent donc un rôle dominant dans l'effet carcinogène de l'exposition solaire. La pigmentation est un facteur intervenant dans la sensibilité constitutive aux UV. Les UVA absorbés par la mélanine des mélanocytes seraient à l'origine des cancers cutanés. En fait, les UVA sont responsables de l'initiation du mélanome, mais les UVB sont aussi responsables de son développement jusqu'à la métastase.

La principale indication du rétinaldéhyde et de l'acide rétinoïque est le traitement de l'héliodermie, qui correspond à un vieillissement cutané, souvent prématuré, généré par les expositions solaires chroniques et/ou prolongées. Les lésions sont directement dépendantes de la dose reçue.

L'oléamide de glycylglycine de formule :

CH₃-(CH₂)₇-CH=CH-(CH₂)₇-CO-NH-CH₂-CO-NH-CH₂-COOH

est un lipo-peptide ou acyl-peptide issu de la condensation de l'acide oléique et du dipeptide glycylglycine.

L'oléamide de glycylglycine est connu pour ses propriétés tensioactives dans des compositions détergentes liquides (US-4 732 690 et JP-59 84 994). Le brevet EP-1 061 897 a pour objet l'utilisation de l'oléamide de glycylglycine pour fabriquer une composition destinée à lutter contre la glycation et l'élastose des fibres protéiques du tissu conjonctif et d'une manière plus générale en stimulant le métabolisme cellulaire.

L'alpha-tocophérol ou vitamine E se trouve à l'état naturel dans de nombreuses plantes, habituellement avec d'autres composés tels que le béta-tocophérol, le gamma-tocophérol ou le delta-tocophérol. L'alpha-tocophérol est essentiellement utilisé pour lutter contre les déficiences en vitamine E, ou comme facteur nutritionnel, notamment pour lutter contre la dégénérescence musculaire. Il est également utilisé comme antioxydant, mais à des doses très spécifiques. Des compositions cosmétologiques contenant ces tocophérols sont déjà commercialisées. Toutefois, il a été démontré que dans des conditions d'oxydations douces, l'application directe de ces antioxydants sur la peau provoque des effets pro-inflammatoires qui sont la conséquence d'une suractivité aux concentrations couramment utilisées. Ainsi plusieurs modifications structurelles de ces tocophérols ont permis de limiter les effets secondaires, permettant de les utiliser dans le traitement ou la prévention d'autres maladies ou dysfonctionnement de la peau. Ainsi, WO 98/51679 décrit des esters de tocophérols dans des compositions cosmétiques ou pharmaceutiques. Il est décrit dans cette demande internationale que ces esters de tocophérols sont à activité anti radicalaire, anti-inflammatoire, favorisant la différenciation des kératinocytes, améliorant l'hydratation cutanée et la finesse du grain de la peau, destinée à prévenir ou à traiter les effets du vieillissement sur la peau, à activité dépigmentante, ou à action anesthésique sur les terminaisons nerveuses cutanées. Le brevet EP 1062223 décrit des précurseurs d'actifs en cosmétologie ou dermatologie, plus particulièrement dans le traitement des maladies de la peau (dermatite atopique, acné, psoriasis). Le principe de l'invention est lié à l'utilisation de la glucocérébrosidase, qui est une enzyme lysosomale présente dans toutes les cellules et donc présente naturellement dans la peau. La glucocérébrosidase hydrolyse le précurseur actif, libérant ainsi la substance biologiquement active. Par ce moyen les effets secondaires sont diminués, voire éliminés. Ce brevet divulgue ainsi l'utilisation de précurseurs glucidiques de delta-tocophérol et plus particulièrement les caractéristiques physico-chimiques et biologiques de delta-tocophérol-glucopyranoside. L'hydrolyse enzymatique par la glucocérébrosidase du delta-tocophérol-glucopyranoside libère lentement le delta-tocophérol, avec une cinétique plus faible qu'un dérivé de référence (4-méthylumbelliféryl glucopyranoside). Cette libération lente permet d'éviter la surconcentration de l'actif delta-tocophérol et donc tout effet inflammatoire lors de l'application du produit. Ainsi la libération lente de l'actif assure une meilleure biodisponibilité de celui-ci dans le milieu cutané et donc une protection plus efficace. Il est en particulier divulgué dans EP 1062223 la très faible activité des glucoconjugués comparée à celle des actifs libres : en particulier, le delta-tocophérol-glucopyranoside possède un pouvoir antioxydant faible par rapport à celui du delta-tocophérol.

Les travaux menés par la demanderesse ont permis de révéler une synergie sur le métabolisme de rétinoïdes dans des kératinocytes avec l'association du rétinaldéhyde et de l'oléamide de glycylglycine.

De façon surprenante, le delta-tocophérol-glucopyranoside en association avec le rétinaldéhyde et l'oléamide de glycylglycine présente une activité dermatologique sur l'ensemble du derme, révélant un effet très marqué pour lutter contre le vieillissement cutané et plus particulièrement contre le vieillissement cutané photo-induit. Ainsi l'association des 3 actifs présente un très large spectre d'action sur de nombreux gènes dont l'expression est modifiée lors du vieillissement photo-induit.

L'objet de l'invention concerne une composition cosmétique ou dermatologique comprenant à titre de principe actif du rétinaldéhyde et de l'oléamide de glycylglycine.

L'objet de l'invention concerne également une composition cosmétique ou dermatologique comprenant à titre de principe actif du rétinaldéhyde et de l'oléamide de glycylglycine, et au moins un véhicule cosmétiquement ou dermatologiquement acceptable.

Selon un autre mode de l'invention, la composition comprend à titre de principe actif du rétinaldéhyde, de l'oléamide de glycylglycine et du delta-tocophéryl-glucopyranoside, et au moins un véhicule cosmétiquement ou dermatologiquement acceptable.

Selon un mode préféré, l'excipient se présente sous une forme destinée à une application topique. L'objet de l'invention est tout particulièrement destiné à une application topique sur des zones cutanées considérées comme fragiles, comme par exemple le visage, le cou, le contour des yeux et des lèvres.

On entend par « véhicule » tout adjuvant ou excipient permettant la fabrication, la conservation ou l'administration de la composition cosmétique ou dermatologique. Tout véhicule acceptable du point de vue cosmétique ou dermatologique, choisi par exemple parmi les excipients couramment utilisés en galénique, peut être utilisé dans la composition selon l'invention.

Avantageusement la composition selon l'invention contient de 0,01 à 1% en poids de rétinaldéhyde, de préférence de 0,015 à 0,1% en poids de rétinaldéhyde par rapport au poids total de la composition.

Selon un mode préféré de l'invention, la composition contient 0,015% en poids de rétinaldéhyde par rapport au poids total de la composition.

Selon un autre mode préféré de l'invention, la composition contient 0,05% en poids de rétinaldéhyde par rapport au poids total de la composition.

Selon encore un autre mode préféré de l'invention, la composition contient 0,1% en poids de rétinaldéhyde par rapport au poids total de la composition.

Avantageusement la composition selon l'invention contient de 0,0001 à 20% en poids d'oléamide de glycylglycine, de préférence de 0,001 à 10% en poids d'oléamide de glycylglycine, de manière préférée de 0,01 à 1% en poids d'oléamide de glycylglycine, de manière plus avantageuse de 0,05 à 0,5% en poids d'oléamide de glycylglycine, par rapport au poids total de la composition.

Selon un mode préféré de l'invention, la composition contient 0,1% en poids d'oléamide de glycylglycine par rapport au poids total de la composition.

Avantageusement la composition selon l'invention contient de 0,001 à 10% en poids de delta-tocophéryl-glucopyranoside, de préférence de 0,01 à 5% en poids de delta-tocophéryl-glucopyranoside, de manière préférée de 0,05 à 0,5% en poids de delta-tocophéryl-glucopyranoside, et de manière encore plus avantageuse de 0,05 à 0,15% en poids de delta-tocophéryl-glucopyranoside par rapport au poids total de la composition.

Selon un mode préféré de l'invention, la composition contient de 0,05 à 0,1% en poids de delta-tocophéryl-glucopyranoside par rapport au poids total de la composition.

Selon un mode préféré de l'invention, la composition selon l'invention contient de 0,01 à 0,1% de rétinaldéhyde, 0,1% d'oléamide de glycylglycine et de 0.05 à 0,1% de delta-tocophéryl-glucopyranoside, par rapport au poids total de la composition.

La composition selon l'invention peut se présenter sous forme d'émulsion huile dans eau (H/E) ou eau dans huile (E/H). Elle peut encore se présenter sous forme de sphérules comme les liposomes, les nanocapsules ou les nanosphères.

Les phases grasses pouvant être utilisées dans l'invention sont :
- les phases grasses solides ou pâteuses telles que les cires d'abeille, cires de candelila, les cires de canauba, de la cire de pétrole (ou cire microcristalline), la paraffine ; et leurs mélanges ;
- les huiles d'origine animale et/ou végétales ; et leurs mélanges ;
- les huiles d'origine synthétique hydrocarbonées, avec plus de 8 atomes de carbone, pouvant être linéaires, ou ramifiés, saturés ou insaturés, telles que le polyisobutylène hydrogéné (huile de Parléam), l'huile de paraffine (ou vaseline, ou Paraffinium Liquidium), les isoparaffines, le limonene, le squalène, le polyisobutène ou l'isooctane ; et leurs mélanges ;
- les huiles formées d'acides gras supérieurs, notamment en C₁₀-C₂₂, tels que l'acide myristique, l'acide palmitique, l'acide stéarique, l'acide béhénique, l'acide oléique, l'acide linoléique, l'acide linolénique ou l'acide isostéarique ;
- les huiles formées d'alcools gras supérieurs, notamment en C₁₀-C₂₂, tels que l'alcool oléique, l'alcool linoléique ou linolénique, l'alcool isostéarique ou l'octyl dodécanol ; et leurs mélanges ;
- les huiles formées d'esters linéaires ou ramifiés, saturés ou insaturés, de formule RCOOR' dans laquelle R représente le reste d'un acide gras supérieur comportant de 7 à 19 atomes de carbone et R' représente une chaîne hydrocarbonée comportant de 3 à 20 atomes de carbone, en particulier les esters en C₁₂-C₃₆, tels que le myristate d'isopropyle, le palmitate d'isopropyle, le stéarate de butyle, le laurate d'hexyle, l'adipate de diisopropyle, l'isononanoate d'isononyle, le palmitate de 2-éthyl-hexyle, le laurate de 2-hexyl-décyle, le palmitate de 2-octyldécyle, le myristate ou le lactate de 2-octyl-dodécyle, le succinate de di(2-éthyl-hexyle), le malate de diidostéaryle, l'hydroxystéarate d'isostéaryl, l'hydroxystéarate d'étylhexyl, éthylhexanoate de cétéaryl ; l'isostéarate d'isostéaryl ; et leurs mélanges ;
- les huiles formées de monoglycérides d'acides carboxyliques en C₁-C₃₀, de diglycérides d'acide carboxylique en C₁-C₃₀, de triglycérides d'acides carboxyliques en C₁-C₃₀, tels que le mono-, di- ou triglycéride de l'acide caprylique et caprique, le mono-, di- et triglycéride de l'acide palmitique, le mono-, di- et triglycéride de l'acide linoléique, le mono-, di- et triglycéride de l'acide stéarique, le mono-, di- et triglycéride de l'acide isostéarique, le mono-, di- et triglycéride de l'acide béhénique, le mono-, di- et triglycéride de l'acide oléique, le mono-, di- et triglycéride de l'acide myristique, le mono-, di- et triglycéride de l'acide linolénique ; et leurs mélanges ;
- les huiles siliconées non volatiles telles que les polydiméthylsiloxanes (PDMS) non volatils ; les polysiloxanes modifiés ; les silicones aminées, ou à groupement hydroxyles, ou fluorés ; les diméthicones ; les triméthicones ; et leurs mélanges. De manière préférée, les diméthicones pouvant être utilisées dans le cadre de la présente invention sont accessibles sous les noms commerciaux VICASIL ® (General Electric Company), DOW CORNING 200 ® (Dow Corning Corporation, DC200), DOW CORNING 225 ®, ou toute autre phase grasse connue de l'homme du métier.

La composition peut également comprendre des agents de conditionnement de la peau. Des exemples d'agents de conditionnement de la peau peuvent comprendre mais ne sont pas limités aux émulsifiants anioniques, cationiques, non ioniques, tels que le lauryl sulfate de sodium, le dioctyl sulfosuccinate de sodium, le stéarate de sodium, l'ester de sorbitan, des acides gras éthoxylés, des alcools gras éthoxylés, trideceth-9 et le PEG-5 éthylhexanoate, et tout émulsifiant et agent de conditionnement connu de l'homme du métier.

Pour les compositions selon l'invention ayant une base alcoolique-aqueuse ou alcoolique, l'utilisation de tout mono-alcool est convenable.

La composition peut comprendre en outre un polyol miscible à l'eau à la température ambiante (25°C) notamment choisi parmi les polyols ayant notamment de 2 à 20 atomes de carbones, de préférence ayant de 2 à 10 atomes de carbone, et préférentiellement ayant de 2 à 6 atomes de carbone, tels que la glycérine, les dérivés de glycol tel que le propylène glycol, le butylène glycol, le pentylène glycol, l'hexylène glycol, le dipropylène glycol, le diéthylène glycol ; les éthers de glycol tels que les alkyls (C₁-C₄) éther de mono-, di- ou tripropylène glycol, les alkyls (C₁-C₄) éthers de mono-, di- ou triéthylène glycol ; et leurs mélanges.

La composition peut comprendre en outre des agents antimicrobiens tels que des conservateurs ou agents antifongiques choisis parmi les alcools, pouvant contenir un ou plusieurs substituants aromatiques, par exemple les phénoxyéthanol comme le 2-phénoxyéthanol, 1-phénoxy-2-propanol, l'alcool benzylique, le 2-hydroxybiphényl, l'urée de l'imidazolidinyle, l'urée du diazolidinyle, l'hydroxyméthylglycinate de sodium, des dérivés halogénés tels que butylcarbamate d'iodopropynyle, le 2-bromo-2-nitropropan-1,3-diol, 2,4,4'-trichloro-2'-hydroxydiphényléther (triclosan), le 3,4,4'-trichlorocarbanilide (triclocarban), le chlorbutanulum, l'alcool 2, 4-dichlorobenzylique, l'urée du N-4-chlorphényl-N'-3,4-dichlorphényle, le 1,2-dibromo-2,4-dicyanobutane, le chloroxylénol, le cétoconazole, l'oxiconazole, le butoconazole, le clotrimazole, l'éconazole, l'énilconazole, le fenticonazole, le miconazole, le sulconazole, le tioconazole, le fluconazole, l'itraconazole, le terconazole, des actifs contenant un ou plusieurs azotes cationiques tels que le chlorure de cétyltriméthylammonium, le chlorure de cétylpyridinium, le chlorure de benzéthonium, le chlorure de diisobutyléthoxyéthyl-diméthylbenzylammonium, le chlorure de diisobutyl-phénoxy-éthoxyéthyl-diméthylbenzylammonium, le chlorure, bromure saccharinate de N-alkyl-N,N-diméthylbenzylammonium, le chlorure de trimétylammonium, l'aluminiumchlorohydroxylacétate de sodium, le chlorure de tricétylméthylammonium, diaminoalkylamide, des acides organiques et leurs sels, tel que l'acide citrique, des agents antimicrobiens insaturés tels que le farnesol, la terbinafine ou la naftifine, des agents aromatiques hétérocycliques tels que le bifonazole, le cloconazole, l'isoconazole, de tout autre agent antimicrobien ou antifongique connu de l'homme du métier et leurs mélanges.

La composition peut également comprendre des agents épaississants ou des agents de modification de la rhéologie, tels que par exemple des uréthanes nonioniques éthoxylés hydrophobiquement modifiés, des acides polycarboxyliques épaississants tels que le copolymère d'acrylates/stréareth-20 méthacrylate, des carbomères, des copolymères d'acrylates et acrylates et acrylates C₁₀-C₃₀ alkyl acrylate réticulés ; et leurs mélanges.

La composition peut également comprendre des acides et des bases permettant d'ajuster la zone de pH de ladite composition. Les bases peuvent être minérales (soude, potasse, ammoniaque) ou organiques telles que la mono-, di- ou triéthanolamine, un aminométhylpropanediol, la N-méthyl-glucamine, les acides aminés basiques comme l'arginine et la lysine ; et leurs mélanges.

Les compositions peuvent en outre comprendre un ou plusieurs autres ingrédients tels que des tampons de pH, des vitamines, des fragrances, et tout autre composé utile bien connu de l'homme du métier.

La composition selon l'invention peut être conditionnée sous forme d'une pommade, d'un spray, d'une lotion, d'un gel, d'une mousse, d'une dispersion, d'un sérum, de masque, de lait corporel ou de crème.

Les compositions selon l'invention peuvent être utilisées en cosmétologie et en dermatologie pour prévenir ou améliorer les peaux ridées et permettent de maintenir la fermeté et l'humidité de la peau.

Les compositions selon l'invention peuvent être utilisées dans des préparations topiques, dans le domaine de la dermatologie ou de la cosmétologie, dans le but de prévenir et/ou de diminuer les rides, de lutter contre le vieillissement cutané photo-induit ou non, de relancer l'activité cellulaire épidermique et dermique, de raffermir la peau, d'augmenter son élasticité, d'augmenter les réserves physiologiques de la peau en vitamine A.

L'invention concerne également l'utilisation cosmétique de la composition selon l'invention pour lutter contre le vieillissement de la peau, et plus particulièrement contre le vieillissement cutané photo-induit.
L'invention concerne également l'utilisation cosmétique de la composition selon l'invention pour augmenter les réserves physiologiques de la peau en vitamine A

L'invention concerne également un procédé cosmétique pour lutter contre le vieillissement cutané photo-induit comprenant l'application sur la peau de la composition selon l'invention.

L'invention concerne aussi un procédé cosmétique pour augmenter les réserves physiologiques de la peau en vitamine A comprenant l'application sur la peau de la composition selon l'invention.

L'invention concerne également une composition selon l'invention pour son utilisation comme médicament dermatologique pour traiter les affections cutanées en liaison avec un déficit des réserves physiologiques de la peau en vitamine A.

La présente invention est illustrée de façon non limitative par les exemples suivants.

### Exemple 1

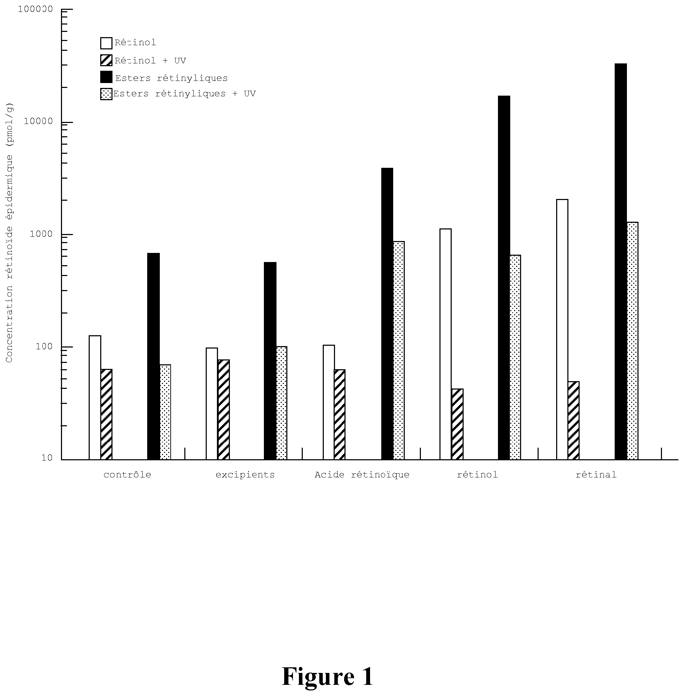

Prévention de la déplétion induite par des rayonnements UV par un prétraitement par rétinoïdes topiques (Tran et al., Photochem. Photobiol. 73(4) : 425-431, 2001).
L'étude est réalisée chez la souris hairless. Les rétinoïdes sont appliqués de façon topique durant 7 jours à 0,05%, puis un rayonnement UVB (1J/cm²) est produit. Les rétinoïdes épidermiques sont dosés 24 h après le rayonnement.
Les résultats sont illustrés dans la figure 1 qui a pour titre : prévention de la déplétion UV induite par un prétraitement par rétinoïdes topiques.
Le rétinaldéhyde et le rétinol préviennent la déplétion photo-induite des esters rétinyliques épidermiques en chargeant l'épiderme en rétinoïdes. L'acide rétinoïque prévient aussi la déplétion photo-induite de rétinoïdes, sans fournir de substrat, mais en induisant la lécithine rétinol acyltransférase. Conclusion : c'est le rétinaldéhyde qui présente le meilleur profil dans cette étude de déplétion de rétinoïdes photo-induite.

### Exemple 2

### Modulation des esters rétinyliques induits par des rétinoïdes dans les kératinocytes

L'étude a été réalisée sur des kératinocytes A431 en culture. En effet, dans ces cellules aucun rétinoïde endogène n'a été mesuré. Il faut appliquer préalablement un rétinoïde pour mesurer des rétinoïdes cellulaires. Les rétinoïdes ont été mesurés par HPLC. L'incubation en présence de différents agents ou combinaison d'agents dissous dans 1% d'éthanol dans le milieu de culture, a été réalisée durant 24 à 72 h.

La production de rétinoïdes dans les kératinocytes est présentée dans le tableau ci-dessous. Il s'agit de valeurs moyennées exprimées en pmol/mg protéine.

**Tableau 1**

| Traitement | Rétinol | Esters rétinyliques | Total |
|---|---|---|---|
| Ethanol (1%) | < 10 | < 10 | < 10 |
| OA (15µM) | < 10 | < 10 | < 10 |
| GG (15µM) | < 10 | < 10 | < 10 |
| OGG (15µM) | < 10 | < 10 | < 10 |
| ROL-Pal (15µM) | 389 | 178 | 567 |
| RAL (2µM) | 865 | 280 | 1145 |
| RAL + OA | 673 | 304 | 977 |
| RAL + GG | 676 | 238 | 914 |
| RAL + OGG | 945 | 484 | 1429 |

| | | | |
|---|---|---|---|
| OA : acide oléique ; GG : glycylglycine ; OGG : oléamide de glycylglycine ; ROL-Pal : palmitate de rétinyle ; RAL : rétinaldéhyde. | | | |

Cette expérience permet d'établir que le rétinaldéhyde induit une série d'esters rétinyliques. Ces esters rétinyliques induits par le rétinaldéhyde sont significativement augmentés par l'oléamide de glycylglycine, montrant une réelle synergie par cette association. Le palmitate de rétinyle est incorporé dans les cellules, puis hydrolysé en rétinol, puis le rétinol formé est estérifié en partie, donnant au final moins de rétinoïdes cellulaires que le rétinaldéhyde.

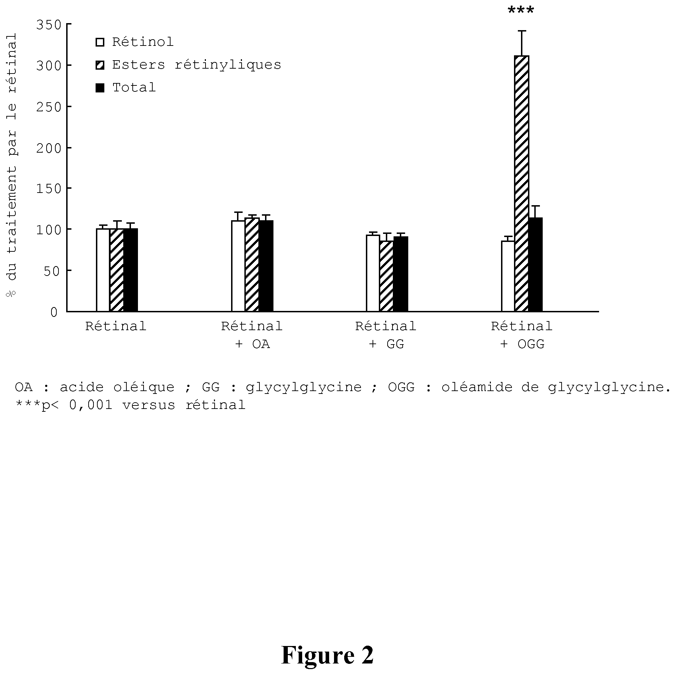

La figure 2 montre la synergie induite par l'association du rétinaldéhyde et de l'oléamide de glycylglycine sur la modulation des esters rétinyliques induits dans les kératinocytes après 48 h d'incubation. Les valeurs sont exprimées en pourcentage du traitement par le rétinaldéhyde.

### Exemple 3

### Effet de l'association du rétinaldéhyde de l'oléamide de glycylglycine et du delta-tocophéryl-glucopyranoside sur des fibroblastes dermiques humains irradiés avec des UVA.

L'objectif de cette étude est d'évaluer les propriétés protectrices de ces trois actifs avant une irradiation des fibroblastes.

L'étude a été réalisée sur des fibroblastes dermiques humains normaux, ensemencés en plaques 12 puits et cultivés en milieu de culture pendant 24 heures.
Pour la recherche d'un effet protecteur, le milieu de culture a été remplacé par un milieu contenant ou non (témoin) le mélange des produits à tester, et les cellules ont été incubées pendant 24 heures. A la fin de la pré-incubation, le milieu a été remplacé par un milieu d'irradiation en absence des composés puis les cellules ont été irradiées ou non (témoin non irradié) par des UVA (20J/cm²). A la fin de l'irradiation, le milieu a été remplacé par un milieu d'essai et les cellules ont été incubées pendant 48 heures. Toutes les conditions expérimentales ont été réalisées en n=3. A l'issue de l'expérience les ARN ont été extraits puis l'expression de 64 gènes a été mesurée par RT-PCR.

Le mélange consiste en :
- rétinaldéhyde à 1µM
- oléamide de glycylglycine à 1µM
- delta-tocophéryl-glucopyranoside à 10µM.

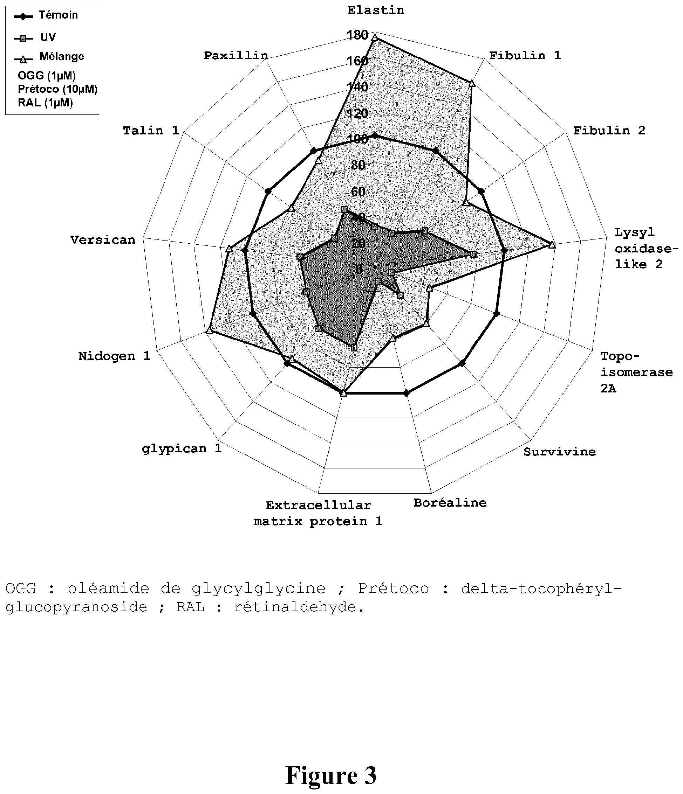

L'irradiation par UVA des fibroblastes dermiques humains a nettement inhibé l'expression de nombreux gènes impliqués dans l'assemblage de la matrice extracellulaire ainsi que l'expression de certains composants de cette matrice (Figure 3). Parallèlement, une nette stimulation des marqueurs MMP1 et MMP3, impliqués dans la dégradation de la matrice extracellulaire a été observée. Enfin, l'expression de nombreux gènes impliqués dans la régulation du cycle cellulaire était inhibée. Sur les 64 gènes testés, 25 ont été régulés par les UV. Ces résultats étaient attendus et ont permis de valider l'expérience.

En présence du mélange des trois actifs, comme le montre la figure 3, un effet protecteur important est observé sur 13 gènes dont l'expression était modifiée par l'irradiation.
De façon surprenante, le mélange de ces trois actifs permet d'observer un effet protecteur aussi bien sur des gènes impliqués dans le réseau élastique de la peau (comme l'elastin, la fibulin 1), dans les interactions entre les cellules et la matrice extracellulaire (comme par exemple paxillin, versican) mais également dans la protection du noyau des cellules (survivin, boréaline).

La figure 3 permet de visualiser que le mélange de ces trois actifs restaure l'expression des gènes affectés par l'irradiation mais également de voir une potentialisation de l'expression de 4 gènes, par rapport aux conditions contrôles (elastine, fibulin, lysyl oxidase-like 2 et nidogen 1).

Ainsi le mélange de ces trois actifs permet non seulement de rectifier les altérations produites par l'irradiation, mais l'effet protecteur dermique est tellement important, notamment sur les gènes impliqués dans l'élasticité de la peau où l'on observe une véritable réorganisation du réseau élastique cutané que le mélange de ces trois actifs est très efficace pour lutter contre le vieillissement cutané.

### Exemple 4

### Effet de l'association de l'oléamide de glycylglycine, du rétinaldéhyde et du delta-tocophéryl-glucopyranoside sur le réseau élastique de la peau humaine irradiée avec des UVA.

L'étude a été réalisée sur des explants de peau humaine ensemencés en plaques 6 puits et cultivés en milieu de culture pendant 48 heures. La peau a été irradiée ou non (témoin non irradié) par des UVA (12J/cm²). Pour la recherche d'un effet protecteur, une formule contenant ou non (témoin placebo) le mélange d'oléamide de glycylglycine à 0.1%, de rétinaldéhyde à 0.05%, et de delta-tocophéryl-glucopyranoside 0.05% a été appliquée à la surface de la peau (10 mg/cm²) juste à la fin de l'irradiation. Après 24 heures d'incubation, la même quantité de formule protectrice a été à nouveau appliquée sur la peau. L'expérience a été prolongée pour 24 heures à l'issue desquelles la peau a été congelée et traitée pour visualiser le réseau élastique du derme cutané par immunohistochimie en microscopie confocale à fluorescence. L'expression de la fibrilline et de l'élastine a été analysée à l'aide d'anticorps spécifiques.

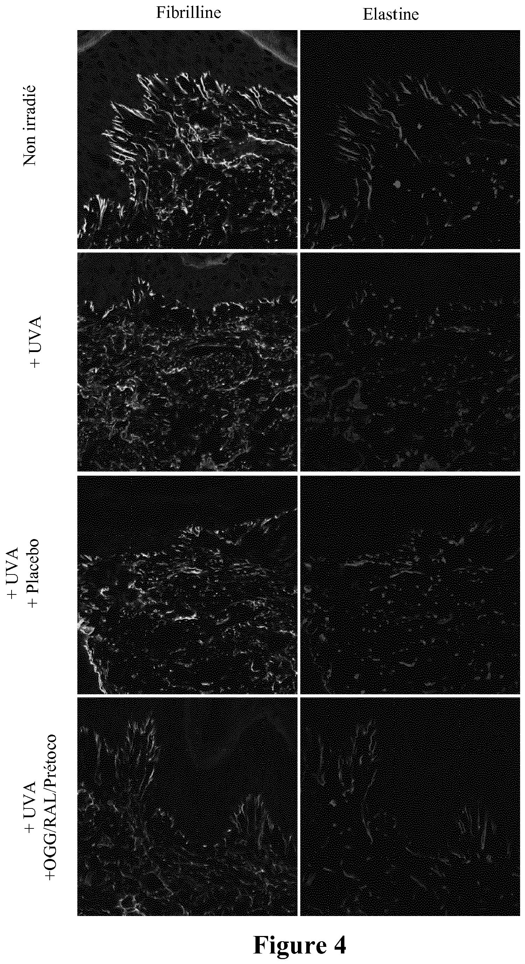

Les résultats sont illustrés dans la figure 4 concerne l'application topique du mélange oléamide de glycylglycine, rétinaldéhyde et delta-tocophéryl-glucopyranoside, protégeant la peau contre les altérations des fibres élastiques induites par l'irradiation UVA. En effet, l'irradiation par UVA des explants cutanés a fortement endommagé l'expression de la fibrilline et de l'élastine dans le derme et a provoqué une désorganisation importante du réseau de fibres élastiques avec de nombreuses cassures. Le témoin placebo n'a pas protégé la peau et a présenté un réseau élastique similaire à la peau irradiée UVA. Par contre, la formule contenant les trois actifs a permis de restaurer la structure des fibres élastiques affectées par les UVA et de protéger le capital élastique de la peau. La structure du réseau élastique de la peau irradiée et traitée avec le mélange d'actifs est identique à celle d'une peau témoin non irradiée.

L'association oléamide de glycylglycine, rétinaldéhyde et delta-tocophéryl-glucopyranoside permet donc de réorganiser le tissu élastique cutané et de protéger la peau contre les altérations générées par les UVA. Ainsi, le mélange des trois actifs préserve le capital élastique cutané et agit efficacement contre le photovieillissement de la peau.

L'ensemble des résultats prouve que l'association du rétinaldéhyde et de l'oléamide de glycylglycine par sa synergie va permettre d'augmenter durablement les réserves physiologiques de la peau en vitamine A. L'association du rétinaldéhyde, de l'oléamide de glycylglycine et du delta-tocophéryl-glucopyranoside va permettre de lutter efficacement contre le vieillissement cutané en agissant en synergie au niveau du derme sur une multitude de cibles.

### Exemple 5 : crème

| | % en poids |
|---|---|
| Oléamide de glycylglyc ine | 0.1 |
| Rétinaldéhyde | 0.1 |
| delta-tocophéryl-glucopyranoside | 0.1 |
| Glycérine | 6 |
| Disodium EDTA | 0.1 |
| Phenoxyéthanol | 0.35 |
| C10-C30AlkylCrosspolymer | 0.4 |
| Glyceryl Stéarate & PEG-100 Stearate | 4 |
| Alcool cétylique | 1 |
| Caprylic/Capric Triglycérides | 10 |
| Diméthicone | 4 |
| Dicaprylyl Carbonate | 4 |
| eau | QSP 100% |

## Revendications

1. Composition cosmétique ou dermatologique comprenant à titre de principe actif du rétinaldéhyde et de l'oléamide de glycylglycine et au moins un véhicule cosmétiquement ou dermatologiquement acceptable,
pour son utilisation comme médicament dermatologique pour traiter les affections cutanées en liaison avec un déficit des réserves physiologiques de la peau en vitamine A.

2. Composition pour son utilisation selon la revendication 1, **caractérisée en ce qu'**elle comprend en outre du delta-tocophéryl-glucopyranoside.

3. Composition pour son utilisation selon l'une des revendications 1 et 2, **caractérisée en ce qu'**elle contient 0,01 à 1% en poids de rétinaldéhyde par rapport au poids total de la composition.

4. Composition pour son utilisation selon l'une quelconque des revendications 1 à 3, **caractérisée en ce qu'**elle contient 0,0001 à 20% en poids d'oléamide de glycylglycine par rapport au poids total de la composition.

5. Composition pour son utilisation selon l'une quelconque des revendications 2 à 4, **caractérisée en ce qu'**elle contient 0,001 à 10% de delta-tocophéryl-glucopyranoside par rapport au poids total de la composition.

6. Composition pour son utilisation selon la revendication 2, **caractérisée en ce qu'**elle contient de 0,01 à 0,1% de rétinaldéhyde, 0,1% d'oléamide de glycylglycine et de 0.05 à 0,1% de delta-tocophéryl-glucopyranoside, par rapport au poids total de la composition.

7. Composition pour son utilisation selon l'une quelconque des revendications 1 à 6, **caractérisée en ce que** l'excipient se présente sous une forme destinée à une application topique.

8. Utilisation cosmétique d'une composition telle que définie dans l'une quelconque des revendications 1 à 7, pour augmenter les réserves physiologiques de la peau en vitamine A.

9. Procédé cosmétique pour augmenter les réserves physiologiques de la peau en vitamine A comprenant l'application sur la peau d'une composition telle que définie dans l'une quelconque des revendications 1 à 7.

## Patentansprüche

1. Kosmetische oder dermatologische Zusammensetzung umfassend als Wirkstoff Retinaldehyd und Glycylglycinoleamid und zumindest einen kosmetisch oder dermatologisch verträglichen Träger
zur Verwendung als dermatologisches Arzneimittel zur Behandlung von Hauterkrankungen im Zusammenhang mit einem Defizit der physiologischen Reserven der Haut an Vitamin A.

2. Zusammensetzung zur Verwendung nach Anspruch 1, **dadurch gekennzeichnet, dass** sie ferner Delta-Tocopheryl-glucopyranosid umfasst.

3. Zusammensetzung zur Verwendung nach einem der Ansprüche 1 und 2, **dadurch gekennzeichnet, dass** sie 0,01 bis 1 % Gewichtsprozent Retinaldehyd im Verhältnis zum Gesamtgewicht der Zusammensetzung enthält.

4. Zusammensetzung zur Verwendung nach einem der Ansprüche 1 bis 3, **dadurch gekennzeichnet, dass** sie 0,0001 bis 20 % Gewichtsprozent Glycylglycinoleamid im Verhältnis zum Gesamtgewicht der Zusammensetzung enthält.

5. Zusammensetzung zur Verwendung nach einem der Ansprüche 1 bis 4, **dadurch gekennzeichnet, dass** sie 0,001 bis 10 % Delta-Tocopheryl-glucopyranosid im Verhältnis zum Gesamtgewicht der Zusammensetzung enthält.

6. Zusammensetzung zur Verwendung nach Anspruch 2, **dadurch gekennzeichnet, dass** sie 0,01 bis 0,1 % Retinaldehyd, 0,1 % Glycylglycinoleamid und 0,05 bis 0,1 % Delta-Tocopheryl-glucopyranosid im Verhältnis zum Gesamtgewicht der Zusammensetzung enthält.

7. Zusammensetzung zur Verwendung nach einem der Ansprüche 1 bis 6, **dadurch gekennzeichnet, dass** der Hilfsstoff in einer für eine topische Anwendung bestimmten Form vorliegt.

8. Kosmetische Verwendung einer Zusammensetzung nach einem der Ansprüche 1 bis 7, um die physiologischen Reserven der Haut an Vitamin A zu vergrößern.

9. Kosmetisches Verfahren zur Vergrößerung der physiologischen Reserven der Haut an Vitamin A, umfassend das Auftragen einer Zusammensetzung auf der Haut, wie sie in einem der Ansprüche 1 bis 7 definiert ist.

## Claims

1. A cosmetic or dermatological composition comprising as an active ingredient retinaldehyde and glycylglycine oleamide and at least one cosmetically or dermatologically acceptable carrier,
for use as a dermatological drug for treating skin diseases in connection with a deficiency in the physiological reserves of the skin in vitamin A.

2. The composition for use according to claim 1, **characterized in that** it further comprises delta-tocopheryl-glucopyranoside.

3. The composition for use according to one of claims 1 and 2, **characterized in that** it contains 0.01 to 1 % by weight of retinaldehyde based on the total weight of the composition.

4. The composition for use according to any one of claims 1 to 3, **characterized in that** it contains 0.0001 to 20 % by weight of glycylglycine oleamide based on the total weight of the composition.

5. The composition for use according to any one of claims 2 to 4, **characterized in that** it contains 0.001 to 10 % of delta-tocopheryl-glucopyranoside based on the total weight of the composition.

6. The composition for use according to claim 2, **characterized in that** it contains 0.01 to 0.1 % of retinaldehyde, 0.1 % of glycylglycine oleamide and 0.05 to 0.1 % of delta-tocopheryl-glucopyranoside, based on the total weight of the composition.

7. The composition for use according to any one of claims 1 to 6, **characterized in that** the excipient appears in a form intended for topical application.

8. The cosmetic use of a composition as defined in any one of claims 1 to 7, for increasing the physiological reserves of the skin in vitamin A.

9. A cosmetic process for increasing the physiological reserves of the skin in vitamin A comprising the application on the skin of a composition as defined in any one of claims 1 to 7.
